# EUROPEAN PATENT SPECIFICATION

(11) **EP 0 680 360 B1**
(45) Date of publication and mention of the grant of the patent: **07.05.1997**
(21) Application number: 94905032.2
(22) Date of filing: 10.01.1994
(51) Int. Cl.: A61M 39/16

(54) **STERILE ENVELOPE FOR MEDICAL APPLIANCES**
KEIMFREIER UMSCHLAG FÜR MEDIZINISCHE GERÄTE
ENVELOPPE STERILE POUR DES INSTRUMENTS MEDICAUX

(30) Priority: 20.01.1993 IT TO930006 U
(43) Date of publication of application: 08.11.1995
(73) Proprietor: FORINO, Luciano, I-10134 Torino (IT); GUGLIOTTA, Vincenzo, I-10128 Torino (IT); ROSSETTI, Giovanni, I-10048 Vinovo (IT)
(72) Inventor: FORINO, Luciano, I-10134 Torino (IT); GUGLIOTTA, Vincenzo, I-10128 Torino (IT); ROSSETTI, Giovanni, I-10048 Vinovo (IT)
(74) Representative: Aprà, Mario
(86) International application number: EP9400065
(87) International publication number: WO9416763

(56) References cited:
- EP-A- 0 311 532
- DE-U- 8 815 624
- US-A- 4 704 177

## Description

The present invention relates to a flexible envelope for protecting medical appliances against contamination, particularly as when used for the administration, draining or withdrawal of substances.

Medical appliances used for the administration to patients and the draining or withdrawal from patients of substances such as fluids, for therapeutic or diagnostic purposes, are at serious risk of contamination by microbes, viruses, bacteria, etc. present in the environment. These contaminating agents can easily be introduced into the abovementioned appliances, e.g. through the tube connections, taps, valves and so forth, with which such appliances are provided, and cause serious infections in the patient using said appliances.

Current hospital practice is to wrap such medical appliances (or parts thereof) in pieces of gauze or bandages impregnated with sterilizing or anticontaminating agents and sometimes to enclose the whole in an impermeable protective film for safety. But this procedure is laborious, impedes quick access to the protected appliance and is not even reliable from the point of view of asepsis; it is therefore often neglected.
EP-A-0,311,532 discloses a flexible protective envelope of the specified type comprising:
a) a backing consisting of a strong, flexible, elastic, impermeable film;
b) a layer of spongy, absorbent material, smaller than the backing and attached centrally thereto;
c) lateral and transverse closure means which have self-retaining hooks and loops (of the type known as "Velcro", registered trademark) with which to close the envelope onto itself, the appliance being held inside, after the envelope has been folded along its central transverse axis.

The layer of spongy material is attached to the backing with hot-melt adhesive. The lateral and transverse closure means are also fixed to the backing with hot-melt adhesive.

However, this flexible protective wrapping has the following principal drawbacks:
- the layer of spongy material which is impregnated during use with sterilizing or anti-contaminating agents in a liquid or pasty state readily releases said agents, which escape through the lateral edges of the envelope and are then lost and get on to other appliances, the operators and the patient;
- the adhesive used to attach the spongy layer and the lateral and transverse closure means to the backing is subject to the chemical action of the sterilizing and anti-contaminating agents, and to the physical action of repeated opening forces, and consequently its properties soon fail and said parts become detached from the backing;
- the so-called "Velcro"-type closure means when closed upon each other provide no protective barrier against contaminating agents, which therefore get inside the closed envelope through the perimeter areas.

The above drawbacks are the starting point of this invention, the object of which is to eliminate the same.

The main object of the present invention is therefore to provide a flexible envelope for protecting medical appliances against contamination, particularly as when used for the administration, draining or withdrawal of substances, that will guarantee anti-bacterial, anti-viral and anti-microbial protection against contamination rising up all kinds of appliances and systems in medical, pharmaceutical and non-drug uses as used for the administration to patients and the sampling and withdrawal from patients of substances (organic or otherwise), e.g. using infusion or withdrawal techniques.

Another object is to provide a protective envelope as specified that will safely hold in its interior the sterilizing and anti-contaminating agents with which it is impregnated; that will not be subject to detachment of parts during use; and that will provide a closure around its external perimeter forming a secure barrier against the ingress of contaminating agents.

A further object is to provide a protective envelope as indicated that will be simple in structure, safe, convenient and reliable in use and simple to manufacture industrially.

To achieve these objects, the present invention provides a flexible envelope for protecting medical appliances against contamination, particularly as when used for the administration, draining or withdrawal of substances, the essential features of which are the subject matter of the main claim, which should be regarded as incorporated here in its entirety.

Further advantageous features are described in the subordinate claims, which should likewise be regarded as incorporated here in their entirety.

The present invention is described in detail below with reference to the accompanying illustrative drawings in which:
- Fig. 1 is a plan view of the flexible protective envelope according to the model, opened out and showing its inner side;
- Figures 2 and 3 are partial sections on an enlarged scale taken through lines II-II and III-III, respectively, shown in Fig. 1;
- Fig. 4 is a schematic perspective view illustrating the flexible protective envelope according to the model wrapped partly round a connection with two taps for flexible tubes, when the envelope is about to be folded over upon itself;
- Fig. 5 is a view similar to that shown in Fig. 4 but with the flexible protective envelope folded fully over upon itself and closed to protect said connection;
- Fig. 6 is a view similar to that shown in Fig. 5 but showing the envelope with the connection inside it suspended from hanging means;
- Fig. 7 is a view similar to that shown in Fig. 1 but illustrating a small version of the envelope according to the present invention.

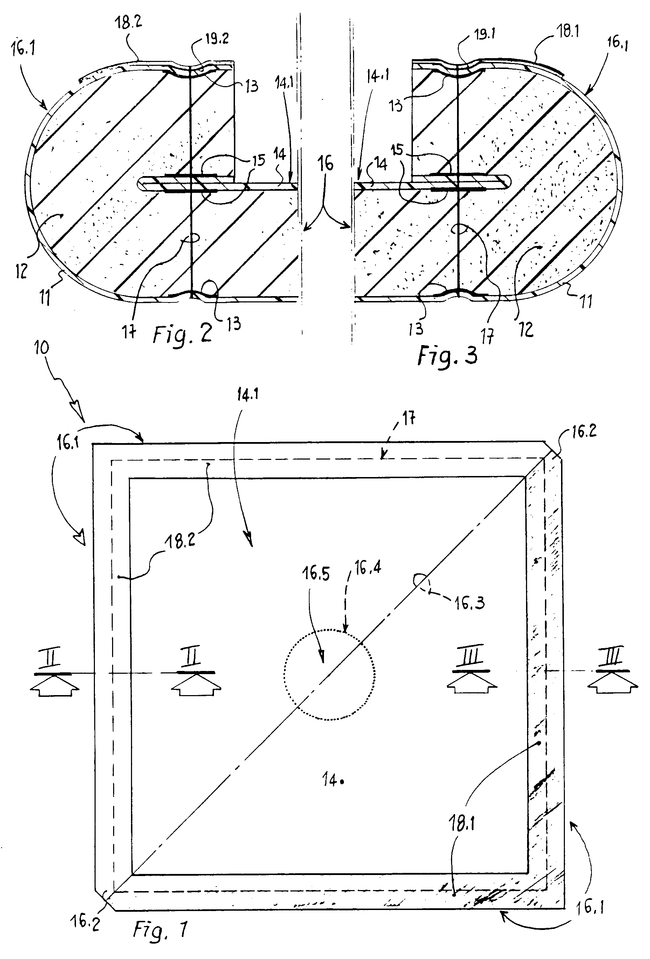

In the drawings the numeral 10 (Fig. 1) denotes the flexible protective envelope as whole according to the present invention.

The envelope 10 essentially comprises the following components (see in particular Figs. 2 and 3):
- a highly impermeable and strong flexible backing sheet 11 consisting of, for example, a flexible polyvinyl chloride (PVC) film having a thickness of 100-150 µm and a square outline, like the embodiment shown in Figures 1-6;
- a layer of padding 12 made of a highly absorbent flexible spongy synthetic material, for example a layer of a flexible expanded polyurethane material such as Moltopren (registered trademark) having a uniform thickness of 3 mm, which entirely covers one side of said backing sheet 11 and is attached to it by a continuous weld line (at 13), e.g. by hot welding or electrical welding;
- a permeable flexible nonwoven covering 14 attached on top of said layer of padding 12 by a continuous weld line (at 15), e.g. by hot welding or electrical welding, and is, for example, cross processed, 20 µm, which components form a pad, for example a quadrilateral pad, in the form of a three-layer sandwich 16, the middle layer being the layer of padding 12.

The peripheral edges of said sandwich pad 16 are turned over on to the free side 14.1 of the covering 14 and are sewn with a continuous through seam 17 (cf. Figs. 2, 3) so as to provide a continuous quadrilateral hem 16.1 in relief around the peripheral edges of said free side 14.1 of the covering 14.

Two opposite external corners 16.2 of said sandwich pad 16 are cut obliquely to help it to fold along an imaginary diagonal line 16.3 joining these corners.

Attached by means of the seam 17 to the outer side of two adjoining edges of said hem 16.1 that lie in the same half relative to the diagonal fold line 16.3, is a corresponding adhesive sealing strip 18.1, in PVC for example, its free side being adhesive. Attached by means of said seam 17 to the outer side of the other two edges of the hem 16.1 is a corresponding sealing strip 18.2, in PVC for example, its free surface being nonadhesive but able to stick to the adhesive strip 18.1 when the envelope 10 is folded over upon itself so as to close it and seal it. Advantageously, said strips 18.1, 18.2 are welded uninterruptedly (at 19.1, 19.2) to the backing sheet 11 by hot welding or electrical welding, at least along said seam 17.

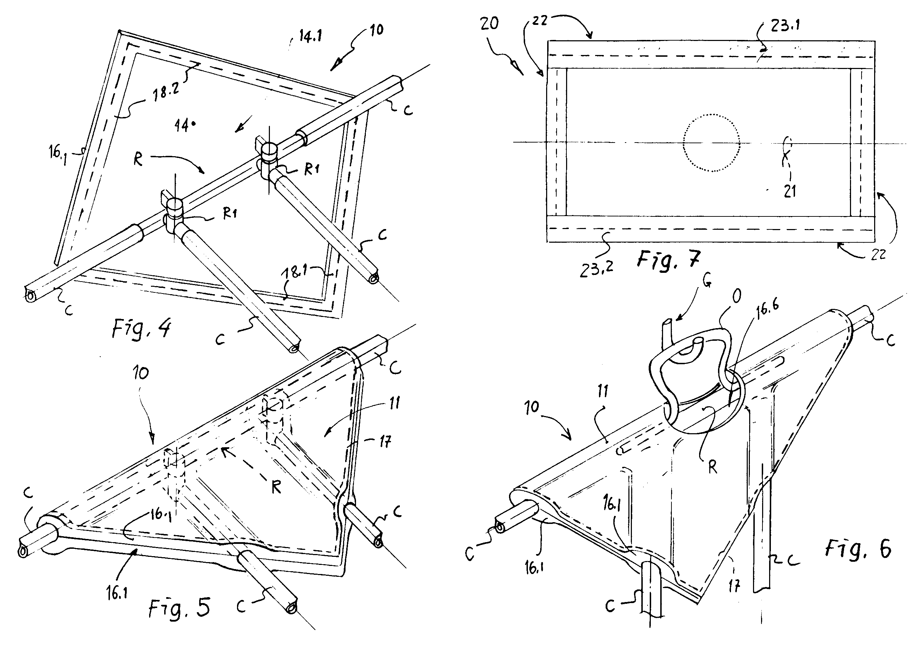

In the middle of said sandwich pad 16 is a microperforated punched circular imprint 16.4 for the selective manual removal of a corresponding discoidal portion 16.5 of said pad 16, in order that a window 16.6 (Fig. 6) can be provided when necessary, as further described below.

For use, the envelope 10 is placed with its side 14.1 uppermost and is impregnated by applying through the covering 14 a suitable sterilizing or anti-contaminating agent, for example an active liquid disinfectant.

The padding 12 absorbs and uniformly imbibes the disinfectant, thereby providing an aseptic field. While the PVC backing sheet 11 prevents the escape of the liquid disinfectant, the nonwoven covering 14 traps the molecules of water released over time by the disinfectant within the Moltopren padding 12. This prolongs the action of the aseptic field created within the protective envelope 10.

A medical appliance or a part of a medical appliance to be protected is then placed on top of said side 14.1 of the envelope 10, arranging it on one half of this side bounded by the diagonal fold line 16.3 (Fig. 4). In Figures 4 to 6 the sketch of the appliance shows a connection R with two taps R1, to each of which a flexible pipe C is connected.

The envelope 10 is then folded over upon itself along said diagonal fold line 16.3 until fully closed, as illustrated in Fig. 5, in the arrangement in which the two edges of the hem 16.1 furnished with the adhesive strip 18.1 are laid over the two respective edges of said hem 16.1 furnished with the nonadhesive strip 18.2. Light hand pressure on said superimposed edges seals the protective envelope 10 along said peripheral hem 16.1 with the appliance R to be protected contained inside. The flexible pipes C of the appliance R emerge from the closed envelope 10 through corresponding passages formed between the superimposed edges of the hem 16.1 and are held firmly in position by the adhesive strip 18.1 which adheres to their surface.

Among the advantageous results of the above-mentioned arrangement are the following:
- the envelope 10 forms a closed chamber in which the appliance R is firmly accommodated and from which the applied liquid disinfectant or antiseptic cannot escape;
- the sealing together of the superimposed edges of the hem 16.1 ensures not only that the liquid agent cannot escape but also that contaminating agents cannot infiltrate;
- furthermore, any contaminating agents that do get into said closed chamber, or into the envelope 10 while still open, cannot penetrate between the sandwiched layers 11, 12, 14 of the pad 16 because of the presence of the continuous welded joins 13, 15, nor between the backing 11 and the sealing strips 18.1, 18.2 because of the continuous weld lines 19.1, 19.2. The only pathway for such agents is the padding 12 and covering 14, and these are the very components that form the active field of asepsis protecting the appliance R;
- the welding together of the components of the pad 16 guarantees a continuous secure join and seal between the layers 11, 12 and 14 (and also 18.1, 18.2 / 11), and permits, for example, the use of disinfectant or antiseptic substances with a high alcoholic content - which would not be possible in the presence of adhesive agents between said layers; and
- when the envelope 10 is opened, the appliance R is kept in position by the adhesive strip 18.1 adhering to the flexible pipes C.

As illustrated in Fig. 6, the appliance R protected inside the envelope 10 can if desired be hung up: it is simply necessary to remove, by hand, the central disc portion 16.5 of the pad 16 and insert into the hole a part with an eyelet O by which it can be hung from a suspended hook G.

Fig. 7 shows an alternative embodiment of the flexible protective envelope according to the model, here denoted as a whole by the numeral 20. The protective envelope 20 differs from the envelope 10 purely in its rectangular form and its smaller area.

The envelope 20 is closed by folding it along its central longitudinal line (indicated by a dot-and-dash line 21) to bring together the longitudinal edges of its peripheral hem 22. Attached to the outer side of one of said longitudinal edges of the hem is an adhesive sealing strip 23.1, of PVC for example, with an adhesive free side, while attached to the outer side of the other longitudinal edge is a nonadhesive sealing strip 23.2, of PVC for example, able to stick to the strip 23.1 to close and seal the envelope 20.

In all other respects the previous description applies.

Clearly, the effects of the present invention extend to models that provide equal utility by using the same innovative concept as expressed in the claims.

Thus, for example, instead of mutually adhesive sealing strips it is possible to use magnetic sealing strips that hold together, separably, by magnetic attraction.

The shape, dimensions and materials etc. can furthermore vary from those described and illustrated purely by way of non-restrictive illustration.

## Claims

1. Flexible envelope for protecting medical appliances against contamination, particularly as when used for the administration, draining or withdrawal of substances comprising:
- a highly impermeable and strong flexible backing sheet (11),
- a layer of padding (12) disposed on the backing sheet (11) made of a highly absorbent flexible spongy material,
- and mutually adhesive but separable means (18.1, 18.2, 23.1, 23.2); characterized in that:
- the layer of padding entirely covers one side of said backing sheet (11) and is attached to it by a weld line (at 13), e.g. by hot welding or electrical welding;
- a flexible nonwoven covering (14) is attached to and covering said layer of padding (12) by a weld line (at 15), for example by hot welding or electrical welding, providing a three-layer sandwich pad (16), the peripheral edges of which are turned over on to the free side (14.1) of said covering (14) and are sewn with a seam (17) so as to form a continuous hem (16.1) in relief,
and the mutually adhesive but separable means (18.1, 18.2; 23.1, 23.2) are sealing means which, when said flexible envelope (10, 20) is closed upon itself by being folded along a predetermined fold line (16.3, 21) adhere separably one on top of the other and close said envelope,
in such a way that, after the middle layer (12) of said envelope has been impregnated with a sterilizing or anti-contaminating agent, and after an appliance (R) that is to be protected against contamination has been placed on said free side (14.1) of the covering (14) in such a way as to occupy a portion thereof bounded by said fold line, the envelope is folded over upon itself along this line until it is closed with the appliance contained and protected inside it.

2. Envelope according to Claim 1, in which said layer of padding (12) is a layer of a flexible expanded polyurethane material such as, for example, Moltopren (registered trademark) having a uniform thickness of approximately 3 mm.

3. Envelope according to Claim 1, in which said flexible nonwoven covering (14) is cross processed, for example 20 µm.

4. Envelope according to Claim 1, characterized in that said separable mutually adhesive sealing means comprise a first sealing strip (18.1, 23.1), in PVC for example, having an adhesive free side attached by said seam (17) to a first part of said hem (16.1), and a second sealing strip (18.2, 23.2), in PVC for example, with a nonadhesive free surface, which latter is attached by said seam (17) to another part of said hem (16.1) opposite the first with respect to said fold line (16.3, 21), and sticks to said first adhesive strip (18.1, 23.1) when the envelope is folded over upon itself so as to close it and seal it.

5. Envelope according to Claim 1, characterized in that said separable mutually adhesive sealing means comprise a first magnetic strip attached to a first part of said hem (16.1), and a second magnetic strip fixed to another part of said hem (16.1) opposite the first with respect to said fold line (16.3, 21), and sticks to said first magnetic strip when the envelope is folded over upon itself so as to close it and seal it.

6. Envelope according to Claim 1, in which said separable mutually adhesive sealing means (18.1, 18.2) are welded (at 19.1, 19.2) to the backing sheet (11) by hot welding or electrical welding.

7. Envelope according to Claim 1, characterized in that it has a microperforated punched imprint (16.4) for the selective manual removal of a corresponding portion (16.5) of the pad (16), in order that a window (16.6) can be provided when necessary, for example for the application of hanging means (O).

8. Envelope according to Claim 1, of an essentially square shape in plan view, characterized in that two opposite external corners (16.2) thereof are cut obliquely to help it to fold along an imaginary diagonal line (16.3) joining these corners.

## Patentansprüche

1. Flexible Hülle zum Schützen medizinischer Geräte gegen Verunreinigung, insbesondere bei Verwendung zur Verabreichung, Drainage oder Entnahme von Substanzen, die folgendes umfaßt:
- einen hochundurchlässigen und starken, flexiblen Träger (11),
- eine auf dem Träger (11) aufgebrachte Lage aus Polsterung (12), die aus einem hochabsorbierenden, flexiblen schwammartigen Material besteht,
- und aneinanderklebende, aber trennbare Mittel (18.1, 18.2, 23.1, 23.2); dadurch gekennzeichnet,
- daß die Polsterlage eine Seite des Trägers (11) völlig bedeckt und mittels einer Schweißnaht (bei 13), zum Beispiel durch Heißschweißen oder elektrisches Schweißen, daran befestigt ist;
- daß eine flexible Umhüllung (14) aus Vliesstoff durch eine Schweißnaht (bei 15), beispielsweise durch Heißschweißen oder elektrisches Schweißen, an der Lage aus Polsterung (12) befestigt ist und diese bedeckt, wodurch ein dreilagiges Verbund-Polster (16) bereitgestellt wird, dessen Umfangsränder auf die freie Seite (14.1) der Umhüllung (14) umgeschlagen und mit einer Naht (17) vernäht sind, so daß ein durchgehender, erhabener Saum (16.1) gebildet wird,
- und daß es sich bei den aneinanderklebenden, aber trennbaren Mitteln (18.1, 18.2; 23.1, 23.2) um Dichtmittel handelt, die, wenn die flexible Hülle (10, 20) durch Falten entlang einer vorbestimmten Falzlinie (16.3, 21) mit sich verschlossen wird, trennbar aufeinanderkleben und die Hülle derart schließen, daß nach Imprägnieren der mittleren Lage (12) der Hülle mit einem Sterilisier- oder Antikontaminiermittel, und nachdem ein gegen Verunreinigung zu schützendes Gerät (R) derart auf der freien Seite (14.1) der Umhüllung (14) plaziert wurde, daß es einen durch die Falzlinie begrenzten Teil davon einnimmt, die Hülle entlang dieser Linie umgefaltet wird, bis sie verschlossen ist, wobei darin das Gerät enthalten ist und geschützt wird.

2. Hülle nach Anspruch 1, bei der es sich bei der Lage aus Polsterung (12) um eine Lage aus einem Polyurethan-Weichschaumstoff wie zum Beispiel Moltropen (eingetragenes Warenzeichen), mit einer gleichmäßigen Dicke von ca. 3 mm handelt.

3. Hülle nach Anspruch 1, bei der die flexible Umhüllung aus Vliesstoff (14) kreuzverarbeitet ist, zum Beispiel 20 µm.

4. Hülle nach Anspruch 1, dadurch gekennzeichnet, daß die trennbaren, aneinanderklebenden Dichtmittel einen ersten Dichtstreifen (18.1, 23.1) aus beispielsweise PVC, wobei eine mit Klebstoff versehene, freie Seite durch die Naht (17) an einem ersten Teil des Saumes (16.1) befestigt ist, und einen zweiten Dichtstreifen (18.2, 23.2) aus beispielsweise PVC, mit einer klebstofffreien freien Fläche, umfassen, wobei letzterer durch die Naht (17) an einem anderen Teil des Saumes (16.1) gegenüber dem ersten bezüglich der Falzlinie (16.3, 21) befestigt ist und an dem ersten Klebstreifen (18.1, 23.1) haftet, wenn die Hülle so auf sich selbst umgefaltet wird, daß sie dicht verschlossen wird.

5. Hülle nach Anspruch 1, dadurch gekennzeichnet, daß die trennbaren, aneinanderklebenden Dichtmittel einen an einem ersten Teil des Saumes (16.1) befestigten ersten Magnetstreifen und einen an einem anderen Teil des Saumes (16.1) gegenüber dem ersten bezüglich der Falzlinie (16.3, 21) befestigten zweiten Magnetstreifen umfassen, der an dem ersten Magnetstreifen haftet, wenn die Hülle so auf sich selbst umgefaltet wird, daß sie dicht verschlossen wird.

6. Hülle nach Anspruch 1, bei der die trennbaren, aneinanderklebenden Dichtmittel (18.1, 18.2) durch Heißschweißen oder elektrisches Schweißen (bei 19.1, 19.2) mit dem Träger (11) verschweißt sind.

7. Hülle nach Anspruch 1, dadurch gekennzeichnet, daß sie zum gezielten manuellen Abtrennen eines entsprechenden Teils (16.5) des Polsters (16), so daß, falls für das Anbringen von Aufhängmittel (0) erforderlich, ein Fenster (16.6) bereitgestellt werden kann, einen mit Mikroperforationen gestanzten Aufdruck (16.4) aufweist.

8. Hülle nach Anspruch 1 mit einer in Draufsicht im wesentlichen quadratischen Form, dadurch gekennzeichnet, daß zwei sich gegenüberliegende Außenecken (16.2) davon schräg geschnitten sind, damit ihr Falten entlang einer gedachten diagonalen Linie (16.3), die diese Ecken verbindet, unterstützt wird.

## Revendications

1. Enveloppe flexible pour protéger des instruments médicaux contre la contamination, en particulier lorsqu'ils sont utilisés pour l'administration, le drainage ou le retrait de substances, comprenant :
- une feuille support flexible très imperméable et robuste (11),
- une couche de rembourrage (12) disposée sur la feuille support (11) en matériau spongieux flexible très absorbant,
- et des moyens mutuellement adhésifs mais séparables (18.1, 18.2, 23.1, 23.2);
caractérisée en ce que :
- la couche de rembourrage recouvre entièrement un côté de ladite feuille support (11) et lui est attachée au moyen d'une ligne de soudure (en 13), par exemple par soudage à chaud ou par soudage électrique;
- un recouvrement flexible non tissé (14) recouvre ladite couche de rembourrage (12) et lui est attaché par une ligne de soudure (en 15), par exemple par soudage à chaud ou par soudage électrique, ce qui produit un coussin en sandwich (16) à trois couches, dont les bords périphériques sont retournés sur le côté libre (14.1) dudit recouvrement (14) et sont cousus avec une couture (17) de façon à former un ourlet continu (16.1) en relief,
et les moyens mutuellement adhésifs mais séparables (18.1, 18.2; 23.1, 23.2) sont des moyens d'étanchéité qui, lorsque ladite enveloppe flexible (10, 20) est refermée sur elle-même en la pliant le long d'une ligne de pliure prédéterminée (16.3, 21), adhèrent de manière séparable l'un sur l'autre et ferment ladite enveloppe,
d'une manière telle qu'après que la couche intermédiaire (12) de ladite enveloppe a été imprégnée d'un agent stérilisant ou anti-contaminant, et après qu'un instrument (R) qui doit être protégé contre la contamination a été placé sur ledit côté libre (14.1) du recouvrement (14) de manière telle qu'il occupe une portion de celui-ci bordée par ladite ligne de pliure, l'enveloppe est repliée sur elle-même le long de cette ligne jusqu'à ce qu'elle soit fermée avec l'instrument contenu et protégé à l'intérieur.

2. Enveloppe selon la revendication 1, dans laquelle ladite couche de rembourrage (12) est une couche de matériau polyuréthane expansé flexible, comme par exemple, du Moltopren (marque déposée) ayant une épaisseur uniforme d'environ 3 mm.

3. Enveloppe selon la revendication 1, dans laquelle ledit recouvrement flexible (14) non tissé est obtenu par traitement croisé par exemple de 20 µm.

4. Enveloppe selon la revendication 1, caractérisée en ce que lesdits moyens d'étanchéité mutuellement adhésifs et séparables comprennent une première bande d'étanchéité (18.1, 23.1) en PVC par exemple, ayant un côté libre adhésif attaché par ladite couture (17) à une première partie dudit ourlet (16.1), et une deuxième bande d'étanchéité (18.2, 23.2), en PVC par exemple, avec une surface libre non adhésive, laquelle est attachée par ladite couture (17) à une autre partie dudit ourlet (16.1) en face de la première par rapport à ladite ligne de pliure (16.3, 21), et colle à ladite première bande adhésive (18.1, 23.1) lorsque l'enveloppe est repliée sur elle-même, de manière à la fermer et à la rendre étanche.

5. Enveloppe selon la revendication 1, caractérisée en ce que lesdits moyens d'étanchéité mutuellement adhésifs et séparables comprennent une première bande magnétique attachée à une première partie dudit ourlet (16.1) et une deuxième bande magnétique fixée à une autre partie dudit ourlet (16.1) en face de la première par rapport à ladite ligne de pliure (16.3, 21) et colle à ladite première bande magnétique lorsque l'enveloppe est repliée sur elle-même, de manière à la fermer et à la rendre étanche.

6. Enveloppe selon la revendication 1, dans laquelle lesdits moyens d'étanchéité mutuellement adhésifs et séparables (18.1, 18.2) sont soudés (en 19.1, 19.2) à la feuille support (11) par soudage à chaud ou par soudage électrique.

7. Enveloppe selon la revendication 1, caractérisée en ce qu'elle a une empreinte poinçonnée microperforée (16.4) pour le retrait manuel sélectif d'une portion correspondante (16.5) du coussin (16), afin qu'une fenêtre (16.6) puisse être prévue si nécessaire, par exemple pour l'application d'un moyen de suspension (O).

8. Enveloppe selon la revendication 1, de forme essentiellement carrée en vue en plan, caractérisée en ce que deux coins externes opposés (16.2) de celle-ci sont coupés obliquement pour faciliter son pliage le long d'une diagonale imaginaire (16.3) joignant ces coins.
